# EUROPEAN PATENT SPECIFICATION

(11) **EP 0 270 248 B1**
(45) Date of publication and mention of the grant of the patent: **16.03.1994**
(21) Application number: 87309704.2
(22) Date of filing: 03.11.1987
(51) Int. Cl.: C12N 15/09, C12N 5/14, A01H 1/00

(54) **DNA useful for the transport of foreign proteins to the plant cell wall**
Für den Fremdproteintransport zur pflanzlichen Zellwand verwendbare DNS
ADN utile pour le transport de protéines étrangères vers la paroi cellulaire végétale

(30) Priority: 11.11.1986 GB 8626878
(43) Date of publication of application: 08.06.1988
(73) Proprietor: ZENECA LIMITED, London W1Y 6LN (GB)
(72) Inventor: Bridges, Ian George, Manley Cheshire (GB); Schuch, Wolfgang Walter, Overton Frodsham Cheshire (GB); Grierson, Donald, Shepshed Leicestershire (GB)
(74) Representative: Roberts, Timothy Wace

(56) References cited:
- EP-A- 0 189 707
- PLANT BIOLOGY, vol. 4, 1987, pages 309-323, Alan R. Liss. Inc.& TOMATO BIOTECHNOLOGY SYMPOSIUM, Davis, CA, 20th-22nd August 1986; D. GRIERSON et al.: "Expression and function of ripening genes"
- PHILOS. TRANS. R. SOC. LONDON B, 1986, vol. 314, no. 1166, pages 399-410, GB; D. GRIERSON et al.: "Gene expression during tomato ripening"
- NUCLEIC ACIDS RESEARCH, vol. 14, no. 21, 11th November 1986, pages 8595-8603; D. GRIERSON et al.: "Sequencing and identification of cDNA clone for tomatopolygalacturonase"
- CHEMICAL ABSTRACTS, vol. 109, 1988, page 400, abstract no. 125987c, Columbus,Ohio, US; P.H. MORGENS et al.: "Searching for molecular mechansisms involved in fruit ripening"
- NATURE, vol. 323, 11th September 1986, pages 178-180; C.M. CONDIT et al.: "Agene encoding a novel glycine-rich structural protein of petunia"

## Description

This invention relates to DNA: and to vectors and plant cells containing such DNA, and to plants composed of such cells.

By the use of known genetic engineering techniques, it is in principle straightforward to modify the biochemistry, and hence the behaviour and properties, of plant cells. This is done by introducing into the cells DNA coding for desired enzymes, in such a manner as to ensure that the foreign DNA is treated by the cell in the same fashion as natural DNA therein. DNA so introduced is expressed: that is to say, the normal mechanisms of the cell transcribe it into messenger RNA, which is thereafter translated into the desired protein (enzyme). Control of the amount of the foreign enzyme found in the cell is possible, eg. by inserting into the foreign DNA promoters that intensify the frequency with which the foreign DNA is transcribed. However, even when expressed in quantity, the foreign enzyme is not necessarily found to have the desired effect in the cell.

The present invention is based on the hypothesis that, in certain cases, failure of the foreign enzyme to have the desired effect arises because the enzyme is not present, or not present in sufficient concentration, in those parts of the cell in which its action is desired.

Certain enzymes are known whose function is to act on plant cell walls. An example is polygalacturonase (hereinafter "PG"). This enzyme is involved in the ripening of climacteric fruit, in particular tomatoes. It is believed that PG causes cell wall softening in tomatoes as a result of degradation of cell wall pectin.

Recent investigations of the structure of PG, and of the messenger RHA from which it is translated, have shown that PG is produced from a longer precursor protein known as "prepolygalacturonase" (PPG - see Sato et al (1984), Plant and Cell Physiology, 25, 1069-71). Mature PG is formed from PPG by loss of 71 amino acid units from the N-terminus (Grierson et al, Nucleic Acid Research, 1986, 14, pp 8595-8603 - see also Grierson et al, Plant Biol.,4 , pp 309-323, an account of a Tomato Biotechnology Symposium held in Davis, CA, USA on 20-22 August 1986). The protein fragment so lost is termed a leader sequence or leader transit peptide.

The present invention bases itself on the hypothesis that the PG leader sequence, and others like it in other enzymes acting in cell walls, act as a means to target their associated enzymes so that they move to or into cell walls: during the transfer process the precursor protein is believed to be converted into the mature and biochemically active enzyme by the removal of the leader sequence. The invention accordingly proposes to use this mechanism to target other proteins to cell walls.

Accordingly, the present invention provides recombinant DNA comprising a reading frame initiator sequence commencing an upstream base sequence followed by a downstream base sequence in register herewith, the upstream sequence coding for a leader peptide capable of acting as a plant cell wall targeting protein, and the downstream sequence coding for a protein not naturally found in plant cell walls

Preferably the leader peptide is the polypeptide leader of PG.

The invention further comprises vectors including DNA according to the invention; methods of making such vectors by cloning of the desired upstream (or downstream) base sequence into an existing vector containing the desired downstream (or upstream) sequence; plant cells incorporating DNA according to the invention; and plants and seeds composed of such plant cells.

The accompanying drawings are provided to assist in understanding the invention. In these:
Figure 1 gives the base sequence of the clone pTOM6,
Figure 2 shows the structure of the clone pWreck1 cTAK1;
Figure 3 shows the structure of the three related clones pTAK1, pTAK2, pTAK3,
Figure 4 shows the structure of the three related clones pMK1, pMK2, pMK3,
Figure 5 shows the structure of the clone pDHC1,
Figure 6 shows schematically how clones according to the invention are produced from pDHC1,
Figure 7 shows the detailed structure of three clones produced according to the invention.

As stated above, while DNA sequences coding for a substantial part of any convenient plant cell wall targeting protein may be used in the present invention, we prefer to use a DNA sequence coding for at least part (and preferably for all) of the PG leader sequence.

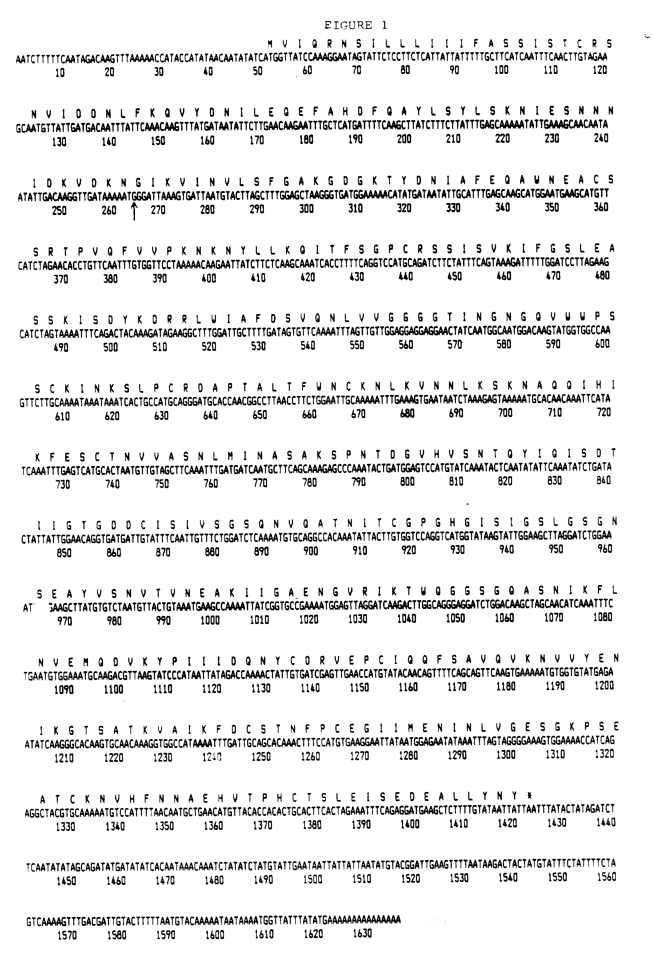

DNA coding for this protein is conveniently obtained as follows. A library of cDNA is prepared from mRNA obtained from ripening tomatoes. This process is described by Slater et al, (1985), Plant Molecular Biology 5, 137-147. In this way may be obtained cDNA sequences coding for the whole or substantially the whole of PPG, or for the whole, substantially the whole or substantial parts of the PG leader sequence. The paper by Grierson et al (of which the present inventors are authors), Nucleic Acid Research 1986, previously referred to, gives the base sequence of pTOM6, a cDNA fragment cloned by Slater et al that corresponds to a substantially complete mRNA molecule found in the production of PPG in the tomato. The sequence is reproduced herein as Figure 1.

The sequence information given in Figure 1 will assist considerably in repeating the work described by Slater et al.

As downstream coding sequence may be used DNA sequences coding for any protein which it is desired to transport to a plant cell wall. Examples of such proteins are fungicidal and insecticidal toxophores, such as chitinases, β-glucanases and Bacillus thuringiensis (BT) toxin. The presence of BT toxin in plant cell walls will serve to protect the plant against insect attack. Chitinase (which may be found in bacteria or plants) attacks chitin, a material found in fungal cell walls and insect exoskeletons. Its presence in plant cell walls may be expected to protect the plant against fungal attack. The downstream sequence may also code for a so-called "marker" gene: one whose expression can be directly detected, thereby revealing its presence. A useful marker gene is the β-glucuronidase (gus) gene (Jefferson et al, Proc. Natl. Acad. Sci. 1986).

Recombinant DNA and vectors according to the present invention may be made as follows. A suitable vector containing the desired upstream base sequence (for example pTOM6) is treated with a restriction enzyme to cut out part or all of the DNA base sequence coding for the cell wall targeting protein. The DNA strand so obtained is cloned into a second vector containing the desired downstream base sequence (for example, a pTAK plasmid -Jefferson, containing the gus gene or derivatives thereof). The restriction enzymes and second vector must be selected so that the first base sequence is introduced into the second vector in the appropriate position: ie. on the immediate 5ʹ side of the second base sequence and in reading frame register therewith. The second vector may usefully contain an upstream promoter sequence, serving to assist expression of the genes below it; or such a promoter sequence may be cloned into it attached to the upstream base sequence at the 5ʹ end. Or a promoter may be included subsequently. A suitable promoter is obtainable from cauliflower mosaic virus (CaMV 35S). The second vector may also include (or subsequently be modified to include) terminator base sequences, whose functions (though not fully understood) appear to be beneficial.

Another way of making vectors according to the invention is to clone the desired downstream base sequence into a suitable vector containing the gene for the cell wall targeting protein, ensuring that the downstream base sequence is introduced at the 3ʹ end of said gene and in reading frame register therewith.

Vectors according to the invention may be used to transform a wide variety of plants, using known techniques. Dicotyledonous plants may be transformed using Ti plasmid technology, as described for example by Bevan (1984), Nucleic Acid Research, 12, 8711-8721. Monocots may be transformed , for example, by injection of the vectors into flowering plants according to the method of Schell, Lörz and de la Pena (Nature, 1987). Transformed plants may be reproduced sexually (giving transformed seed), vegetatively or by cell culture.

Stable homogeneous lines containing the desired gene can be produced by repeated self-pollination and selection for the desired gene. Such lines will find use directly, and in producing F1 hybrids.

Specific embodiments of the invention are further illustrated in the following Examples and Experiments.

These describe the isolation of two PG base leader sequences (one coding for the whole of the PG leader sequence, the other for part of it), their incorporation into plasmids containing the gus marker gene, and the use of such plasmids to transform tobacco. The Examples describe the making and use of plasmids according to the invention, while the Experiments describe the preparation of starting materials.

The sequence of PG cDNA clone pTOM6 (NCIB Accession No. 12351) is shown in Figure 1. The arrow indicates the start of the mature PG protein. This has been determined by direct amino acid sequence determination of the N-terminal amino acids of mature PG protein isolated from ripe tomatoes. The PG leader sequence is represented by the first 71 amino acids encoded by the open reading frame found in pTOM6. This leader sequence is characterised by the presence of a hydrophobic region of about 20 amino acids which is followed by two distinct regions of hydrophilicity. The PG leader sequence is isolated from pTOM6 cDNA clones by digestion of the cDNA with PstI and RsaI. This yields a fragment which comprises most of the 5ʹ untranslated region of the PG mRNA, and encodes the complete PG leader sequence including the first six amino acids of the mature PG protein.

Part of the PG leader sequence can be excised from pTOM6 by restriction of Pst1 and HindIII. This fragment contains the PG 5ʹ untranslated region and 49 amino acids of the PG leader sequence, including the hydrophobic region represented by the first 20 amino acids.

All cloning procedures are performed under standard conditions as described by Maniatis et al (1982) "Molecular Cloning", Cold Spring Harbor Laboratory.

Vectors for which an NCIB Accession number are given have been deposited at the National Collections of Industrial and Marine Bacteria, Torry Research Station, PO Box 31, 135 Abbey Road, Aberdeen Ab9 8DG, Scotland. The dates of deposit were 7 November 1986 (NCIB 12351-5) and 16 October 1987 (NCIB 12567).

### EXPERIMENT 1

### Construction of the plasmid pHP1

### A. Isolation of the nos 3' end.

10µg of pWRECK2-CAMV-CAT-1 (NCIB Accession No 12352) is digested with PvuI in order to linearise the DNA, under conditions recommended by the manufacturer. The completeness of digestion is analysed by running an aliquot of the reaction on 0.8% agarose gel. The reaction is stopped by extraction with phenol/chloroform. DNA is precipitated with ethanol and dried under vacuum. The cohesive ends are removed by incubation of the linearised DNA with T4 polymerase at 37°C for 30 minutes. The enzyme is inactivated by incubation at 65°C for 15 minutes. The reaction volume is increased by the addition of HindIII buffer and HindIII enzyme is added. The reaction is carried out for 2 hours at 37°C. The 250 bp PvuI/HindIII fragment is isolated from agorose gels by electroelution. DNA is phenol/chloroform extracted, precipitated with ethanol and resuspended in water.

### B. Linearisation of pUC18

2µg of pUC18 (commercially available plasmid) DNA is digested with SphI under conditions recommended by the manufacturer. The reaction is stopped by extraction with phenol/chloroform. Cohesive ends are removed by treatment with T4 polymerase for 30 minutes at 37°C. The buffer volume is increased, and HindIII is added. The mixture is incubated for 2 hours at 37°C. The reaction is stoppped by extraction with phenol/chloroform. DNA is precipitated with ethanol and resuspended in water at 100ng/µl.

### C. Cloning of nos 3' end into pUC18 to give pNOS1

1µl of pUC18 prepared under (B) is ligated with 100ng of nos 3' end prepared under (A) in a total of 15µl in the presence of T4 ligase. Incubation is carried out for 24 hours at 16°C. An aliquot of the ligation is transformed into competent TG2 cells. An aliquot of the transformation mix is plated onto ampicillin and Xgal containing plates. White colonies are picked, and the DNA examined by restriction analysis. Molecules containing the nos 3'end are characterised by the presence of a 260 base pair HindIII/BamHI fragment. This plasmid is called pNOS1.

### D. Preparation of the CaMV 35S promoter

The CaMV promoter is obtained by digestion of pWRECK2-CAMV-CAT-1 with ScaI for 2 hours at 37°C. An aliquot of the restriction digest is analysed by electrophoresis on agarose gel. The reaction is stopped by extraction with phenol/chloroform. After ethanol precipitation and resuspension in water, the DNA is cut with HphI for 2 hours at 37°C. The cohesive ends are removed by treatment with T4 polymerase under standard conditions. The CaMV promoter 629 base pair fragment is isolated by agarose gel electrophoresis and subsequent electroelution.

### E. Linearisation of pNOS1

2µg of pNOS1 is cut with SstI at 37°C for 2 hours. After completion of the reaction, T4 polymerase is added in order to remove the cohesive ends. The reaction is stopped by extraction with phenol/chloroform. The DNA is precipitated with ethanol and resuspended in water at 100 ng/µl.

### F. Cloning of CaMV 35S promoter into pNOS1

pNOS1 prepared as under (E) is ligated to CaMV 35S promoter fragment prepared under (D) under standard conditions using T4 ligase. The reaction is carried out for 24 hours at 16°C. An aliquot of the ligation mixture is transformed into competent TG2 cells, and plated onto ampicillin containing Xgal plates. DNA is isolated from transformants and analysed by restriction with NcoI and HindIII. Molecules containing the CaMV 35S promoter in the correct orientation are characterised by the presence of a 920 base pair fragment. This plasmid is called pHP1.

### EXPERIMENT 2

### A. Construction of pMQ-CaMV-1

The CaMV 35S promoter which is used to drive the expression of the PG leader-gus fusion product is isolated from pHP1 (prepared as in Experiment 1) by restriction with EcoRI and PstI. This fragment is then cloned into plasmid pAT153 (commercially available, eg. from Amersham) cut with EcoR1 and Pst1. Recombinants are identified by virtue of the inactivation of the ampicillin resistance gene and restriction analysis followed by Southern hybridisation to the isolated CaMV promoter fragement. This vector is called pMQ-CaMV-1.

### B. Construction of pMQ1-3

The intermediate vector from A, pMQ-CaMV-1, is restricted with HindIII and Pst1 under standard conditions. The resulting CaMV promoter fragment, now on a HindIII to Pst1 fragment, is cloned into the plasmids pTAK 1-3 (Jefferson, NCIB Accession Nos 12353, 12354 and 12355 respectively) which have been cut with HindIII and Pst1 under standard conditions. The required recombinants are identified by filter hybridisation to the isolated CaMV promoter fragment. The correctness of the constructions is confirmed by restriction mapping and direct DNA sequence determination. These vectors are called pMQ1, pMQ2 and pMQ3.

### EXPERIMENT 3

### Isolation of the complete PG leader sequence

5µg of pTOM6 (NCIB Accession No 12351) are digested with PstI under standard conditions. The ends of the cDNA insert are labelled using a cordecypin-labelling kit (Amersham). After purification of the labelled DNA by phenol/chloroform extraction, ethanol precipitation and resuspension of the DNA in water, the DNA is treated with Rsal restriction enzyme under standard conditions. The reaction mix is analysed on a polyacrylamide gel, and the 295 base pair fragment is isolated representing DNA containing the 5' untranslated region of the PG mRNA and the complete PG leader sequence including the first six amino acids of the mature PG polypeptide.

### EXPERIMENT 4

### Isolation of part of the PG leader sequence

5µg of pTOM6 DNA is restricted under standard conditions using HindIII. The reaction is stopped by phenol/chloroform extraction and precipitation of the DNA using ethanol. The "sticky" ends generated by HindIII are filled in using DNA polymerase Klenov fragment under standard conditions. The DNA is extracted with phenol chloroform, precipitated with ethanol, and resuspended. This DNA is now treated with PstI restriction enzyme, under standard conditions. When the reaction reaches completion, the 210 base pair fragment containing the desired part of the PG leader sequence is isolated from polyacrylamide gels.

### EXAMPLE 1

### Construction of pMQ-PG1

The DNA fragment isolated in Experiment 3 is inserted into the gus expression vector, pMQ1. This vector, prepared as in Experiment 2, is first cut with PstI and SmaI restriction enzyme under standard conditions. The expected recombinant is identified using the filter hybridisation screening method of Grunstein and Hogness following standard protocols. The presence of the PG leader sequence is confirmed using digestion with EcoRI and PstI as indication for insertion of the PG leader sequence. This plasmid is called pMQ-PG1.

### EXAMPLE 2

### Construction of pMQ-PG10

pMQ3, prepared as in Experiment 2, is restricted with PstI and SmaI restriction enzymes under standard conditions. After completion of the reaction, the DNA is purified by phenol/chloroform extraction and ethanol precipitation. The DNA fragment containing part of the PG leader sequence (obtained as described in Experiment 4) is cloned into this vector essentially as described in Example 1. The desired recombinant is identified by filter hybridisation using the fragment to be cloned as radioactively labelled probe. The resulting recombinant is called pMQ-PG10.

### EXAMPLE 3

### Transformation of Tobacco leaf disks using pMQ-PG1 and pMQ-PG10.

The expression cassettes pMQ-PG1 and pMQ-PG10 containing the CaMV promoter, PC leader and the gus reporter gene, (prepared as described in Examples 1 and 2 above) are then transferred to tobacco plants using the leaf disk transformation method essentially as described by Bevan et al (EMBO Journal 4, 1921-1926, 1985). After tripartite matings and transfer of the expression cassettes to Agrobacterium strains, sterile leaf segments from plants cultured in vitro are incubated with the bacteria. After incubation for 24 hours, the bacteria are killed by selection on carbenicillin containing media. After 14-28 days, developing shoots are transferred to rooting media. Plantlets are then analysed for the presence of the expression cassettes by Southern hybridisation. Plants containing the expression cassettes are then used for analysis.

### EXAMPLE 4

### Analysis of transformed callus from plants obtained in Example 3.

Developing callus, after leaf disk transformation, is screened fluorimetrically for the presence of the gus reporter gene as proposed by Jefferson.

### EXAMPLE 5

### Analysis of transformed tobacco plants (as obtained in Example 3).

Transformed plants containing pMQ-PG1 and pMQ-PG10 are analysed for the presence of the gus gene product. As controls pMQ1-3 transformed plants are used (expression cassettes without the PG leader sequence).
There follows a second series of Experiments and Examples which have been carried out according to the same general scheme.

### EXPERIMENT 10

### Construction of the plasmids pMK1, pMK2, pMK3

### A. Isolation of CaMV Promoter

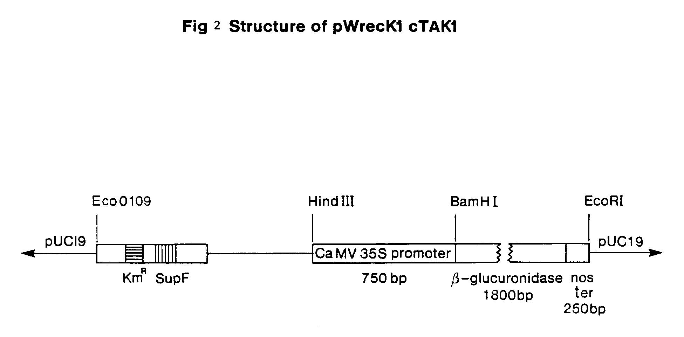

10µg of pWreck1 cTAK1 (Figure 2: NCIB Accession No 12567) was digested with BamH1 and HindIII (0.03M Tris pH 7.4.0.005M MgC1₂, 0.001M DTT, 0.1M NaC1). Completion of digestion was checked after 2 hours at 37°C by running an aliquot on a 0.7% agarose gel. The whole reaction mixture was run on a 0.7% agarose gel and the 750 bp CaMV promoter fragment isolated by electroelution from the excised band. The fragment was extracted with phenol/chloroform and the DNA precipitated with ethanol at -70°C. The fragment was resuspended in water to a concentration of 100ng/µl. The CaMV promoter is available from several alternative sources (see for example Experiment 1).

### B. Preparation of pTAK1, pTAK2 and pTAK3 for Cloning

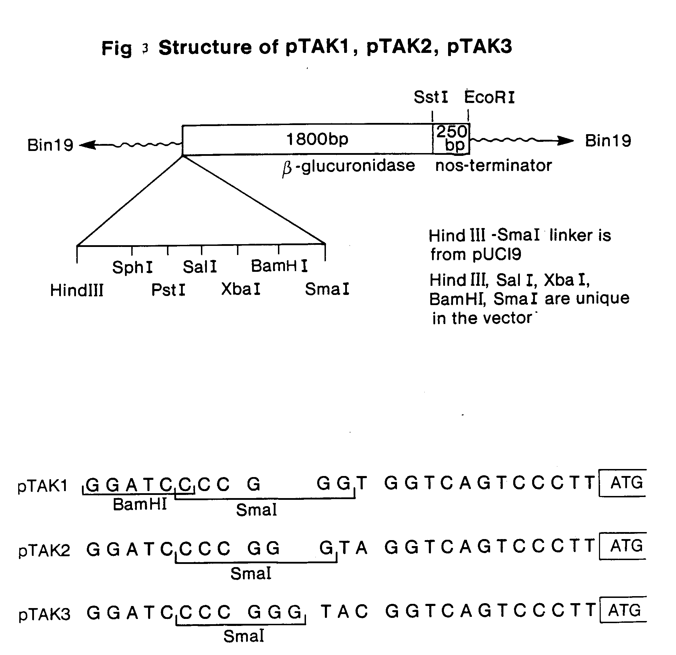

10µg pTAK1, pTAK2 and pTAk3 (Figure 3) were digested with HindIII and BamHI. Completion of digestion was checked by running an aliquot on a 0.7% agarose gel. The reaction was stopped by extraction with phenol/chloroform. The DNA was precipitated with ethanol, then dried in vacuo. The DNA was resuspended in water to a concentration of 100 ng/µl.

### C. Cloning of CaMV Promoter into pTAK1, pTAK2, pTAK3 to give pMK1, pMK2 pMK3

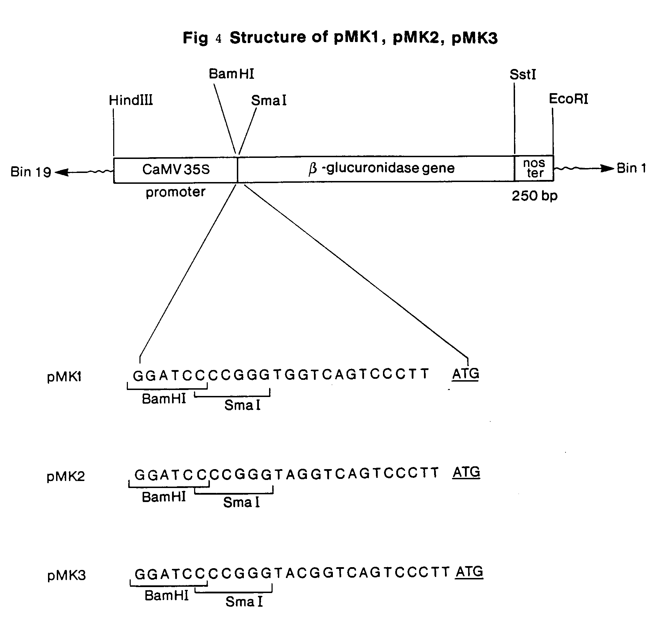

1µl of HindIII-BamHI cut pTAK1, pTAK2 and pTAK3 were ligated with 2 µ1 of CaMV promoter DNA in a total of 10µl in the presence of T4 ligase. Incubation was at 16°C for 24 hours. An aliquot of the ligation mix was used to transform competent TG2 cells. An aliquot of the transformation mix was plated onto kanamycin-containing L-plates (25 mg/cm³), and incubated overnight at 37°C. The required recombinants were identified by filter hybridisation to the isolated CaMV promoter fragment. Hybridising clones were confirmed by restriction digestion with HindIII and BamHI which excises the CaMV promoter on a 750 bp fragment, and DNA sequence analysis. The appropriate recombinants were named pMK1, pMK2 and pMK3 (Figure 4).

### D. Linearisation of pMK1, and pMK3

10 µg pMK1 and pMK3 were digested with SmaI under appropriate conditions. Digestion was checked by running an aliquot of reaction mixture on a 0.7% gel, against uncut DNA. The reaction was terminated by phenol/chloroform extraction. After ethanol precipitation and drying in vacuo the DNA was resuspended in water to a concentration of 100ng/µl.

### EXPERIMENT 11

### Construction of PG leader sequence plasmids

### A. Isolation of the BamHI-HindIII presequence fragment

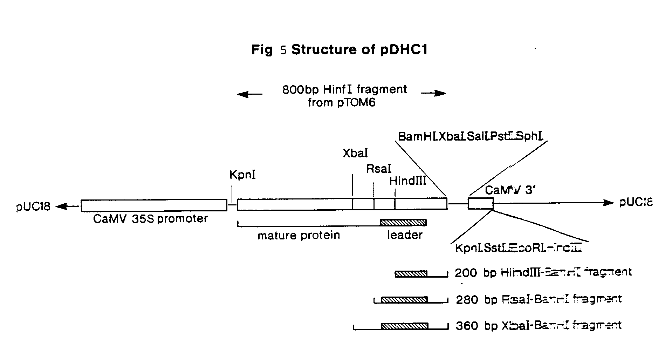

10 µg pDHC1 (Figure 5) was digested with HindIII and BamHI under the conditions of Experiment 10A, above. Completion of digestion was checked by running an aliquot on a 1.5% agarose gel. Digestion of pDHC1 with HindIII leads to two fragments, one of which is the vector, the other is 400bp in length. Cutting the latter with BamHI gave two 200bp fragments which were indistinguishable. Two fragmetns, each 200 bp in size were isolated by electroelution. After phenol extraction, the DNAs were ethanol precipitated after drying resuspended in 40 1 H₂0. The fragments were filled in using Klenow fragment (room temperature 15 mins, 65°C 10 mins) phenol extracted and ethanol precipitated. The DNA was resuspended in 20 µ1 water.

### B. Isolation of RsaI-BamHI presequence fragment

10µg pDHC1 (Figure 5) was digested with RsaI and BamHI under appropriate conditions. Completion of digestion was checked by running an aliquot on a 1.5% agarose gel. The 280 bp fragment was electroeluted from an excised gel slice. After phenol extraction, the DNA was ethanol precipitated and after drying in vacuo resuspended in 40µl H₂0. The fragment was filled in using Klenow fragment. After ethanol precipitation the DNA was resuspended in 20µl H₂0.

### C. Isolation of XbaI-BamHI presequence fragment

10µg pDHC1 (Figure 5) was digested with XbaI and BamHI under appropriate conditions. Digestion was checked for completion by running an aliquot on a 1.5% agarose gel. The 360 bp fragment was isolated by electroelution from the excised gel slice. After precipitation the DNA was resuspended in 40µl H₂0. The cohesive ends were filled in using Klenow fragment as described earlier. After ethanol precipitation, the DNA was resuspended in 20µl H₂0.

### EXAMPLE 10

### D. Cloning of XbaI-BamHI presequence fragment and RsaI-BamHI presequence fragments into pMK1

1µl of restricted pMK1 was ligated separately with 3 µl of the XbaI-BamHI and RsaI-BamHI fragment with T4 ligase at 16°C for 24 hours. The ligation mixes were diluted 5 times and 1µl of the diluted volume used to transform DH5α competent cells (BRL). Aliquots of transformation mix were plated on plates containing kanamycin at 25µg/cm³. The required recombinants were identified by filter hybridisation to the isolated XbaI-BamHI presequence fragment. 180 clones were screened for the presence of the XbaI-BamHI fragment - two positives were identified. 200 clones wee screened for the presence of the RsaI-BamHI fragment - eight positives were identified.

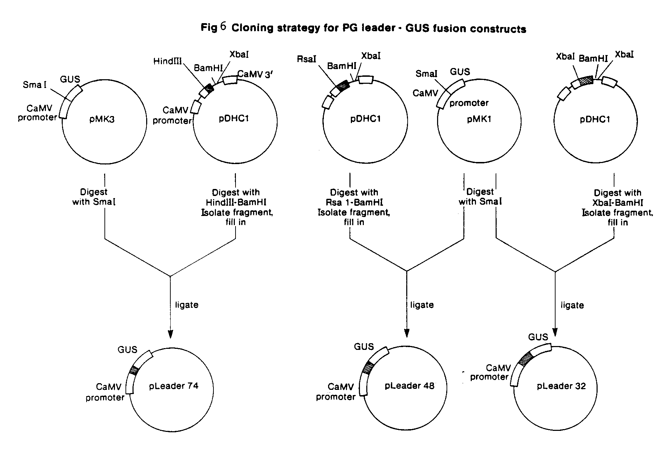

The two clones from the XbaI-BamHI cloning and the eight clones from the RsaI-BamHI cloning were digested with HindIII under appropriate conditions and also with HindIII-BamHI under appropriate conditions. The DNA was transferred to "Gene Screen Plus" using the method of Southern, and the filter probed using labelled oligo CL158 in order to distinguish between the two possible orientations of the insert. One clone containing the XbaI-BamHI fragment in the desired orientation was obtained. One clone containing the RsaI-BamHI fragment in the desired orientation was obtained.
These cloning experiments are summarised in Figure 6.

### EXAMPLE 11

### Cloning of HindIII-BamHI presequence fragment into pMK3.

1 µl restricted pMK3 was ligated with 3µl HindIII-BamHI (blunted) fragment at 16°C for 24 hours. The ligation mixes were diluted 5 times and 1 µl was used to transform DH5α competent cells (BRL). Aliquots of transformation mix were plated onto plates containing kanamycin at 25µg/cm³. The required recombinants were identified by filter hybridisation to the isolated XbaI-BamHI fragment.

Four hundred clones were screened for the presence of the HindIII-BamHI fragment; 12 positives were identified.

These cloning experiments are summarised in Figure 6.

### EXPERIMENT 12

### Sequence Analysis of clones

The putative clones in the correct orientation (1 XbaI-BamHI and 1 RsaI-BamHI from Example 10 and 12 HindIII-BamHI from Example 11) were digested with HindIII and SnaBI in buffer containing 50mM NaC1. Fragments containing the CaMV 355 promoter, the presequence and 350 bp of GUS gene were cloned into HindIII-SmaI cut M13 mp9. Single stranded templates were prepared from these clones and sequenced by the dideoxynucleotide chain termination method. Oligo CL321 which hybridises at the 5' end of the GUS gene was used to prime the sequencing reaction. Sequencing confirmed the correct reading frame and orientation of the constructs containing the XbaI-BamHI fragment (named pLeader 48) and the HindIII-BamHI fragment (named pLeader 74).

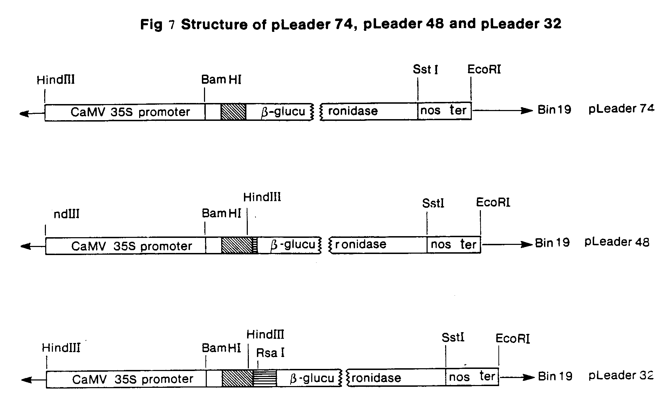

The structure of these clones is shown in Figure 7.

### EXAMPLE 12

### Transformation of tobacco with PG leader sequence constructs.

### A. Transfer of pLeader 32, pLeader 48 and pLeader 74 to Agrobacterium

The recombinant vectors were mobilised from E. coli (TG-2) onto Agrobacterium tumefaciens (LBA4404) in a triparental mating on L-plates with E. coli (HB101) harbouring pRK2013. Transconjugants were selected on minimal medium containing kanamycin (50µg/cm³) and streptomycin (500µg/cm³).

### B. Preparation of Agrobacterium

L-Broth (5cm³) containing kanamycin at 50µg/cm³ was inoculated with a single bacterial colony. The culture was grown overnight at 30°C with shaking at 150 rpm. This culture (500µl) was inoculated into L-Broth containing kanamycin (50µg/cm³) and grown as before. Immediately before use the Agrobacteria were pelleted by spinning at 3000 rpm for 5 minutes and resuspended in an equal volume of liquid Murashige and Skoog (MS) medium.

### C. Preparation of Nicotiana tabacum var Samsun leaf pieces.

Feeder plates were prepared in 9cm diameter petri dishes as follows. Solid MS medium supplemented with 6-Benzyl-aminopurine (6-BAP) (1mg/l) and 1-Naphthaleneacetic acid (NAA) (0.1 mg/l) was overlaid with Nicotiana tabacum var Samsun suspension culture (1 cm³). One 9cm and one 7cm filter paper disc were placed on the surface. Whole leaves from tissue culture grown plants were placed in the feeder plates. The plates were sealed with "Nescofilm" and incubated overnight in a plant growth room (26°C under bright fluorescent light).

### D. Transformation Protocol

Leaves from the feeder plates were placed in Agrobacteria suspension in 12cm diameter petri dishes and cut into 1 - 1.5 cm² sections. After 20 minutes the leaf pieces were returned to the feeder plates which were sealed and replaced in the growth room. After 48 hours incubation in the growth room the plant material was transferred to MS medium supplemented with 6-BAP (1 mg/1), NAA (0.1 mg/1), carbenicillin (500µg/cm³) and kanamycin (100µg/cm³), in petri dishes. The petri dishes were sealed and returned to the growth room.

Beginning three weeks after inoculation with Agrobacterium, shoots were removed from the explants and placed on MS medium supplemented with carbenicillin (200µg/cm³) and kanamycin (100µg/cm³) for rooting. Transformed plants rooted 1-2 weeks after transfer.

**TABLE 1**

| CONSTRUCT PIECES | NUMBER OF LEAF |
|---|---|
| pLeader 48 | 158 |
| pLeader 32 | 126 |
| pMK1 | 139 |
| pMK2 | 128 |

The following numbers of transformed plants have been regenerated:

**TABLE II**

| VECTOR | NUMBER OF TRANSFORMED PLANTS | NUMBER OF PLANTS EXPRESSING GUS |
|---|---|---|
| pMK1 | 10 | 9 |
| pMK2 | 11 | 7 |
| pLeader 32 | 13 | 2 |
| pLeader 48 | 18 | 8 |

Plants were analysed for the expression of GUS marker gene using a fluorometric assay.

Plants expressing GUS were also analysed using cytological methods in order to determine the location of the GUS enzyme.

Experiments are in hand to show that transformed plants expressing the gus gene locate it preferentially in plant cell walls.

## Claims

1. Recombinant DNA comprising a reading frame initiator sequence commencing an upstream base sequence followed by a downstream base sequence in register herewith, the upstream sequence coding for a leader peptide capable of acting as a plant cell wall targeting protein fragment, and the downstream sequence coding for a protein not naturally found in plant cell walls.

2. DNA as claimed in claim 1 in which the leader peptide is the leader protein associated with prepolygalacturonase.

3. DNA as claimed in claim 2 in which the leader peptide has the 71 amino-acid sequence shown in Figure 1 from base 51 to base 263.

4. DNA as claimed in any of claims 2 to 4 in which the upstream base sequence includes a substantial part of the sequence of bases numbered from 51 to 263 in Figure 1.

5. DNA as claimed in claim 4 having an upstream base sequence continuing beyond base 263 and terminating at the RsaI restriction site at bases 282-285 of Figure 1.

6. DNA as claimed in claim 4 having an upstream base sequence terminating at the HinDIII restriction site at bases 195-201 of Figure 1.

7. DNA as claimed in any of claims 1 to 6 in which the downstream sequence codes for the protein β-glucuronidase.

8. DNA as claimed in any of claims 1 to 6 in which the downstream sequence codes for an insecticidal or antifungal protein.

9. Recombinant DNA comprising a reading frame initiator sequence commencing an upstream base sequence followed by a downstream base sequence in register therewith, the upstream sequence coding for the leader peptide of prepolygalacturonase, and the downstream sequence coding for an insecticidal or antifungal protein selected from Bacillus thuringiensis toxin, chitinase and β-glucanase.

10. A vector containing DNA as claimed in any of claims 1 to 9.

11. A vector as claimed in claim 10 which is a plasmid.

12. A vector as claimed in claim 11 which is derived from a pTAK plasmid deposited with the National Collection of industrial and Marine Bacteria under the accession numbers NCIB 12353, 12354 and 12355 respectively.

13. A process for making a vector claimed in any of claims 10 to 12 which comprises cutting with a suitable restriction enzyme the upstream base sequence from a first vector containing it and cloning it into a second vector containing the downstream sequence, adjacent to, upstream of, and in reading frame register with said downstream sequence.

## Patentansprüche

1. Rekombinante DNA, die eine Leseraster-Initiatorsequenz aufweist, die einer flußaufwärts angeordneten Basensequenz vorgeschaltet ist, an die sich damit im Takt eine flußabwärts angeordnete Basensequenz anschließt, wobei die flußaufwärts angeordnete Basensequenz für ein Leader-Peptid kodiert, das in der Lage ist, als Zielmarkierungs-Proteinfragment für eine Pflanzenzellwand zu wirken, und die flußabwärts angeordnete Sequenz für ein Protein kodiert, das nicht natürlich in Pflanzenzellwänden vorkommt.

2. DNA nach Anspruch 1, bei der das Leader-Peptid das mit Prepolygalacturonase assoziierte Leader-Protein ist.

3. DNA nach Anspruch 2, bei der das Leader-Peptid die 71 Aminosäuren lange Sequenz aufweist, die in Figur 1 von der Base 51 bis zur Base 263 dargestellt ist.

4. DNA nach irgendeinem der Ansprüche 2 bis 4, bei der die flußaufwärts angeordnete Basensequenz einen wesentlichen Teil der Sequenz der mit den Nummern 51 bis 263 in Figur 1 bezeichneten Basen einschließt.

5. DNA nach Anspruch 4 mit einer flußaufwärts angeordneten Basensequenz, die sich über die Base 263 hinaus erstreckt und an der RsaI-Restriktionsstelle an den Basen 282-285 von Figur 1 endet.

6. DNA nach Anspruch 4 mit einer flußaufwärts angeordneten Basensequenz, die an der HindIII-Restriktionsstelle an den Basen 195-201 von Figur 1 endet.

7. DNA nach irgendeinem der Ansprüche 1 bis 6, bei der die flußabwärts angeordnete Sequenz für das Protein β-Glucuronidase kodiert.

8. DNA nach irgendeinem der Ansprüche 1 bis 6, bei der die flußabwärts angeordnete Sequenz für ein insektizides oder fungizides Protein kodiert.

9. Rekombinante DNA, die eine Leseraster-Initiatorsequenz aufweist, die eine flußaufwärts angeordnete Basensequenz einleitet, an die sich damit im Takt eine flußabwärts angeordnete Basensequenz anschließt, wobei die flußaufwärts angeordnete Basensequenz für das Leader-Peptid der Prepolygalacturonase kodiert, und die flußabwärts angeordnete Sequenz für ein insektizides oder fungizides Protein kodiert, das ausgewählt ist aus dem Bacillus thuringiensis-Toxin, Chitinase und β-Glucanase.

10. Ein Vektor, der eine DNA enthält, wie sie in irgendeinem der Ansprüche 1 bis 9 beansprucht wird.

11. Ein Vektor nach Anspruch 10, der ein Plasmid ist.

12. Ein Vektor nach Anspruch 11, der sich von einem pTAK-Plasmid ableitet, das bei der National Collection of Industrial and Marine Bacteria unter der Hinterlegungsnummer 12353, 12354 bzw. 12355 hinterlegt ist.

13. Verfahren zur Herstellung eines Vektors nach einem der Ansprüche 10 bis 12, das das Schneiden mit Hilfe eines geeigneten Restriktionsenzyms der flußaufwärts angeordneten Basensequenz aus einem ersten Vektor, der diese enthält, und deren Klonieren in einen zweiten Vektor einschließt, der die flußabwärts angeordnete Sequenz enthält, und zwar daran anschließend, flußaufwärts dazu und im Leseraster-Takt mit der flußabwärts angeordneten Sequenz.

## Revendications

1. ADN recombinant comprenant une séquence d'initiateur cadre de lecture commençant par une séquence de bases amont suivie par une séquence de bases aval concordant avec ladite séquence, la séquence amont codant pour un peptide leader capable de jouer le rôle de fragment de protéine d'acheminement vers une cible de paroi cellulaire d'un végétal, et la séquence aval codant pour une protéine non présente à l'état naturel dans les parois cellulaires de végétaux.

2. ADN suivant la revendication 1, dans lequel le peptide leader est la protéine leader associée à la prépolygalacturonase.

3. ADN suivant la revendication 2, dans lequel le peptide leader possède la séquence de 71 amino-acides représentée sur la figure 1, de la base 51 à la base 263.

4. ADN suivant l'une quelconque des revendications 2 et 3, dans lequel la séquence de bases amont comprend une partie importante de la séquence de bases numérotées de 51 à 263 sur la figure 1.

5. ADN suivant la revendication 4, ayant une séquence de bases amont se continuant au-delà de la base 263 et se terminant au site de restriction RsaI au niveau des bases 282-285 de la figure 1.

6. ADN suivant la revendication 4, ayant une séquence de bases amont se terminant au site de restriction HindIII au niveau des bases 195-201 de la figure 1.

7. ADN suivant l'une quelconque des revendications 1 à 6, dans lequel la séquence aval code pour la protéine appelée β-glucuronidase.

8. ADN suivant l'une quelconque des revendications 1 à 6, dans lequel la séquence aval code pour une protéine insecticide ou antifongique.

9. ADN recombinant comprenant une séquence d'initiateur en cadre de lecture, commençant par une séquence de bases amont suivie par une séquence de bases aval concordant avec ladite séquence, la séquence amont codant pour le peptide leader de la prépolygalacturonase, et la séquence aval codant pour une protéine insecticide ou antifongique choisie entre la toxine de Bacillus thuringisis, la chitinase et la β-glucanase.

10. Vecteur contant un ADN suivant l'une quelconque des revendications 1 à 9.

11. Vecteur suivant la revendication 10, qui est un plasmide.

12. Vecteur suivant la revendication 11, qui est dérivé d'un plasmide pTAK déposé dans la National Collection of Industrial and Marine Bacteria sous les numéros de dépôt respectifs NCIB 12353, 12354 et 12355.

13. Procédé de production d'un vecteur suivant l'une quelconque des revendications 10 à 12, qui comprend l'excision avec un enzyme de restriction convenable de la séquence de bases amont d'un premier vecteur contenant cette séquence et le clonage de ladite séquence dans un second vecteur contenant la séquence aval, en une position adjacente à, en amont de, et en cadre de lecture avec, ladite séquence aval.
